# FASCICULE DE BREVET EUROPEEN

(11) **EP 1 485 066 B1**
(45) Date de publication et mention de la délivrance du brevet: **21.09.2016**
(21) Numéro de dépôt: 03725281.4
(22) Date de dépôt: 12.03.2003
(51) Int. Cl.: A61K 9/06

(54) **COMPOSITION A PROPRIETES GELIFIANTES DESTINEE A LA DELIVRANCE PROLONGEE DE SUBSTANCES BIO-ACTIVES**
ZUSAMMENSETZUNG MIT GELIERENDEN EIGENSCHAFTEN ZUR VERLÄNGERTEN FREIGABE BIOAKTIVER SUBSTANZEN
COMPOSITION HAVING GELLING PROPERTIES FOR THE PROLONGED DELIVERY OF BIOACTIVE SUBSTANCES

(30) Priorité: 12.03.2002 FR 0203059; 26.08.2002 US 405720 P
(43) Date de publication de la demande: 15.12.2004
(73) Titulaire: Ethypharm, 92210 Saint-Cloud (FR); Université de Montréal, Montréal, Québec H3C 3J7 (CA)
(72) Inventeur: LEROUX, Jean Christophe, Montreal, Quebec (CA); COUFFIN-HOARAU, Anne-Claude, Montreal, Quebec H2X 3H7 (CA)
(74) Mandataire: Regimbeau
(86) Numéro de dépôt international: PCT/FR2003/000797
(87) Numéro de publication internationale: WO 2003/075885

(56) Documents cités:
- EP-A- 1 063 007
- WO-A-99/13913
- WO-A-99/56725
- FR-A- 2 281 162
- MURDAN S ET AL: "NOVEL SORBITAN MONOSTEARATE ORGANOGELS" JOURNAL OF PHARMACEUTICAL SCIENCES, AMERICAN PHARMACEUTICAL ASSOCIATION. WASHINGTON, US, vol. 88, no. 6, juin 1999 (1999-06), pages 608-614, XP000825429 ISSN: 0022-3549
- X. LUO ET AL.: "Self-assembled organogels formed by mono-chain L-alanine derivatives" CHEMICAL COMMUNICATIONS, vol. 2001, no. 17, 7 septembre 2001 (2001-09-07), pages 1556-1557, XP002220130 Cambridge (GB)
- S. BHATTACHARYA ET AL.: "First report of phase selective gelation of oil from oil/water mixtures. Possible implications toward containing oil spills" CHEMICAL COMMUNICATIONS, vol. 2001, no. 2, 21 janvier 2001 (2001-01-21), pages 185-186, XP002220131 Royal society of chemistry (GB)
- HANABUSA K ET AL: "ORGANOGELS FORMED BY N-BENZYLOXYCARBONYL-L-ALANINE 4-HEXADECANOYL-2-NITROPHENYL ESTER AND RELATED COMPOUNDS", JOURNAL OF COLLOID AND INTERFACE SCIENCE, ACADEMIC PRESS, NEW YORK, NY, US LNKD- DOI:10.1006/JCIS.1997.5139, vol. 195, no. 1, 1 January 1997 (1997-01-01), pages 86-93, XP008071791, ISSN: 0021-9797

## Description

La présente invention est telle que définie dans les revendications. Elle concerne notamment une composition liquide thermosensible à propriétés gélifiantes à titre de médicament injectable dans un organisme vivant par voie parentérale extravasculaire, intramusculaire, intradermique, intrapéritonéale, intra-oculaire, intracavitale, par voie vaginale, sur une plaie ouverte ou lors d'une intervention chirurgicale, contenant :
- un liquide organique hydrophobe, choisi parmi :
   ∘ l'huile de soja, le squalène, le benzoate de benzyle, le chlorure de benzyle, un mélange de benzoate de benzyle et d'alcool benzylique,
   ∘ les esters d'acides gras du glycérol,
   ∘ ou leur mélange,
- une substance organogélatrice choisie parmi : le N-lauroyl L-alanine méthyle ester, le N-lauroyl L-alanine éthyle ester, le N-stéaroyl L-alanine méthyle ester, le N-stéaroyl L-alanine éthyle ester, et le N-lauroyl L-alanine acide dont les molécules ont la capacité de se lier entre elles par liaisons de faible énergie, et
- une substance bioactive,
qui passe sous forme d'organogel lorsqu'elle entre en contact avec un liquide physiologique, lors de son administration à un corps animal, en particulier l'homme, ledit organogel restant sous forme de gel *in vivo* à la température corporelle.
Ladite composition est destinée à être administrée à un organisme vivant, pour la délivrance prolongée de substances bioactives.

Ladite composition a la capacité de former un organogel de façon spontanée ou par refroidissement, une fois mise au contact d'un milieu aqueux, et notamment, un liquide physiologique. Ledit organogel formé sert de support à la libération prolongée de substances bioactives par diffusion et/ou par érosion et/ou biodégradation progressive dudit organogel dans l'organisme.

La présente description s'étend également aux utilisations qui peuvent être faites de cette composition dans le domaine thérapeutique et plus particulièrement dans le domaine de la délivrance prolongée de substances bioactives.

On entend par composition thermosensible, toute composition capable de passer de l'état liquide à l'état gel en fonction de la température et par organogel tout gel dont la phase liquide est composée par un solvant organique.

Par ailleurs, on entend par substance bioactive, toute substance ayant la capacité d'agir sur un organisme vivant ou son fonctionnement de façon à prévenir, guérir, soulager ou améliorer l'état dudit organisme.

On entend par liquide organique hydrophobe, un solvant ou un mélange de solvants organique(s) dont les molécules ou les parties de molécules présentent une certaine répulsion vis-à-vis des molécules d'eau. On entend par solvant hydrophile, un solvant dont les molécules établissent des interactions d'attraction avec les molécules d'eau.

Les gels sont depuis longtemps utilisés dans le domaine de l'industrie pour les propriétés que leur confère leur structure physique particulière.

En effet, ils correspondent à un état intermédiaire de la matière car ils sont composés à la fois d'éléments sous forme solide et d'éléments sous forme liquide. Les éléments solides formant une structure tridimensionnelle ou matrice, organisée en réseau de molécules interconnectées entre elles, ce réseau immobilisant les éléments présents sous forme liquide.

On peut classer les gels en fonction du type de liaisons qui relient entre elles les molécules de la phase solide ou en fonction du type de solvant, organique ou aqueux qui compose la phase liquide.

On appelle hydrogels, les gels dont la phase liquide est une phase aqueuse, pour les différencier des organogels dont la phase liquide est une phase organique.

Les gels dont la matrice est constituée de molécules liées entre elles par des liaisons covalentes sont généralement dans un état stable et irréversible une fois formés. A l'inverse, les gels dont la matrice solide est obtenue par des liaisons de faible énergie (type liaisons hydrogène ou liaisons de Van der Waals notamment), sont généralement des gels réversibles c'est-à-dire pouvant passer de l'état gel à l'état liquide en fonction des conditions environnantes (pH, température, force ionique etc.).

Dans le cas des gels thermosensibles, la température à laquelle est observé le changement d'état est appelée température de transition. Dans le cas particulier des systèmes présentant un comportement d'hystérèse, la température de transition gel/liquide est différente de la température de transition liquide/gel.

Ainsi, les gels sont notamment utilisés dans l'industrie pharmaceutique, pour leur capacité de rétention vis-à-vis de molécules bioactives, notamment dans le cadre d'une administration de substances actives par voie transcutanée.

Cette propriété de rétention a par ailleurs été exploitée pour une utilisation des gels comme vecteurs de délivrance prolongée de médicaments.

Ainsi, le brevet US N° 3,932,624 décrit l'utilisation d'un hydrogel destiné à la délivrance retard de saralasine. Dans ce brevet, le gel est réalisé à base de gélatine, qui, diluée dans une solution de sérum physiologique contenant la substance bioactive va s'en imprégner pour former une structure gélifiée qui pourra être implantée dans l'organisme par voie chirurgicale au niveau sous cutané. Le gel implanté libère de façon progressive la substance active qu'il contient par érosion progressive dudit gel.

Cependant, ce type d'utilisation nécessite d'implanter *in situ* par voie chirurgicale, un gel préalablement formé. Cette opération reste donc à la fois coûteuse et contraignante pour le patient.

Pour pallier cet inconvénient, des hydrogels se formant *in situ* ont été développés.

Ainsi, récemment, la demande de brevet US N° 20010007673 décrit l'utilisation d'un hydrogel se formant *in vivo* destiné à la délivrance retard de molécules bioactives, notamment de protéines. Une composition à base de polymère hydrophile incluant l'alginate, d'un ion métallique polyvalent et de la substance active désirée, est injectée sous forme liquide et passe sous forme gel une fois placée dans l'organisme. De plus, de par la nature trixotrope de la composition à l'état gel, il est possible d'injecter la composition à l'état gel, par exemple à partir d'une seringue par application d'une certaine pression, après quoi la composition retourne à l'état de gel dans l'organisme. Cet hydrogel permet une diffusion retardée de la substance bioactive dans les liquides de l'organisme.

Par ailleurs, le brevet US n° 5 575 815 décrit l'administration intracavitale, i.e. intra-artérielle ou intraveineuse, d'une composition liquide aqueuse qui se transforme ou se viscosifie en hydrogel in vivo. L'utilisation de ce gel pour l'incorporation de substances actives est prévue, notamment pour l'angioplastie. Les hydrogels utilisés sont constitués de polymères polyéthers.

Le brevet US n° 6 344 488 décrit la formation d'un gel contrôlé par la température et dépendant du pH, comprenant un mélange aqueux chitosan/ sel d'organophosphate. L'addition d'un sel mono-phosphate dibasique de polyol ou de sucre à des solutions aqueuses de chitosan conduit à une gélification contrôlée par la température et dépend du pH. Les médicaments sont incorporés audit gel avant la gélification. Les solutions de chitosan/sel d'organophosphate sont stockées à basses températures sous forme de solution et gélifiant in situ après injection sous cutanée, intrapéritonéale ou intramusculaire suite à une augmentation de la température. L'hydrogel ainsi formé peut être utilisé pour la libération de principes actifs.

La demande de brevet WO 97/15287 décrit un système et une méthode pour l'administration parentérale (intramusculaire, intrapéritonéale, sous-cutanée) de médicament dans une matrice polymère biodégradable à un animal à sang chaud sous forme de liquide résultant en la formation d'un dépôt de gel, pour la libération contrôlée du médicament. Le liquide est une solution aqueuse dans laquelle est dissoute ou dispersée une quantité efficace de médicament contenu dans une matrice de bloc copolymère biodégradable. Le copolymère a une température de gélification inverse inférieure à la température du corps de l'animal auquel il est administré et est fait d'un bloc polymère hydrophobe et d'un bloc polymère hydrophile.

La demande de brevet WO 99/56725 décrit une composition pharmaceutique fluide ayant la capacité de gélifier instantanément en présence d'une phase aqueuse, et comprenant, outre une substance active, 3 à 55% en poids de phospholipide, 16 à 72 % en poids d'un ou plusieurs solvants et 4 à 52 % en poids d'un acide gras.

La demande de brevet WO 99/13913 décrit une composition pour la libération prolongée de principes actifs, comprenant un liquide hautement visqueux (HVLCM) insoluble dans l'eau et non polymérique, et optionnellement une substance active.

La demande de brevet EP 1 063 007 décrit une émulsion cosmétique sous forme de gel comprenant une phase huileuse dispersée dans une phase glycolique contenant un tensioactif non ionique apte à former une phase lamellaire au contact de la phase glycolique, la phase glycolique contenant de la glycérine et au moins un glycol.

Murdan et al, Journal of pharmaceutical sciences, 1999 décrit des organogels thermoréversibles préparés à partir de sorbitan monostéarate ou de sorbitan monopalmitate.

D'autres solutions aqueuses thermogélifiantes sont décrites dans la littérature. Parmi celles-ci, on retrouve les solutions de poloxamers (Johnston, T.P. et al., Inulin disposition following intramuscular administration of an inulin/poloxamer gel matrix, J. Parent. Sci Technolo., vol. 43, 279, 1989 ; Johnston, T.P. et al., Sustained delivery of interleukin-2 from a poloxamer 407 gel matrix following intraperitoneal injection in mice, Pharm. Res. 9, 425, 1992; Pec et al., Biological activity of urease formulated in poloxamer 407 after intraperitoneal injection in the rat, J. Pharm. Sci. Vol.81, 626, 1992) et les solutions de xyloglucan (Miyazaki, S. et al., Thermally reversible xyloglucan gels as vehicles for rectal drug delivery., J. Controlled Release, vol 56, 75, 1998).

Cependant, le principal inconvénient des hydrogels réside dans leur faible efficacité relative à la délivrance sur de longues périodes de temps de substances bioactives hydrophiles. Cela est dû notamment à leur importante proportion en eau qui leur confère une forte porosité, conduisant les substances bioactives hydrophiles présentes dans de tels gels à être relativement vite éliminées dans la circulation. Ce phénomène de diffusion est particulièrement important pour des molécules de petite taille très hydrophiles telles que certains médicaments hydrophiles ou certains peptides hydrophiles par exemple. L'efficacité de la libération prolongée de ces substances s'en trouve donc réduite.

Le but de la présente invention est de fournir une nouvelle composition pharmaceutique ayant la capacité de former un organogel permettant la libération sur de longues périodes de temps de substances actives.

De plus, le but de la présente invention est également de fournir un tel support de libération retardée à la fois biocompatible et biodégradable permettant en outre d'être administré sous forme liquide, c'est-à-dire de façon aisée, rapide et peu coûteuse.

Les organogels ont déjà été utilisés comme support permettant la libération retardée de principe actif.

La présente description a pour objet un organogel hydrophobe généré in vivo après avoir été appliqué sous forme liquide. De tels gels ont déjà été décrits dans l'art antérieur.

La demande de brevet n° WO 94/08623 divulgue un organogel hydrophobe contenant de la lécithine et un solvant de la lécithine hydrophobe utilisé pour la libération retardée de protéine. Le gel se forme in vivo, à partir d'une solution injectée en intramusculaire ou en sous cutané, par absorption d'eau à partir du milieu interstitiel lors de l'injection.

Au contraire, l'organogel hydrophobe de la présente invention ne se forme pas par absorption de l'eau environnante.

La présente invention concerne une composition liquide thermosensible à propriétés gélifiantes à titre de médicament injectable dans un organisme vivant par voie parentérale extravasculaire, intramusculaire, intradermique, intrapéritonéale, intra-oculaire, intracavitale, par voie vaginale, sur une plaie ouverte ou lors d'une intervention chirurgicale, contenant:
-- un liquide organique hydrophobe choisi parmi :
   - l'huile de soja, le squalène, le benzoate de benzyle, le chlorure de benzyle, un mélange de benzoate de benzyle et d'alcool benzylique,
      ∘ les esters d'acides gras du glycérol,
      ∘ ou leur mélange,
-- une substance dite organogélatrice choisie parmi : le N-lauroyl L-alanine méthyle ester, le N-lauroyl L-alanine éthyle ester, le N-stéaroyl L-alanine méthyle ester, le N-stéaroyl L-alanine éthyle ester, et le N-lauroyl L-alanine acide dont les molécules ont la capacité de se lier entre elles par liaisons de faible énergie, et
- une substance bioactive,
qui passe sous forme d'organogel lorsqu'elle entre en contact avec un liquide physiologique, lors de son administration à un corps animal, en particulier l'homme, ledit organogel restant sous forme de gel *in vivo* à la température corporelle.

La substance organogélatrice est constituée de molécules capables de se lier entre elles par des liaisons de faible énergie si bien que l'auto-assemblage de ces molécules est avantageusement thermoréversible.

La composition thermosensible sous forme liquide selon l'invention contient un liquide organique hydrophobe, une substance organogélatrice dont les molécules ont la capacité de se lier entre elles par liaisons de faible énergie, et une substance bioactive. Elle passe sous forme d'organogel lorsqu'elle entre en contact avec un liquide physiologique, lors de son administration à un corps animal, en particulier l'Homme, en particulier lors de l'injection dans l'organisme, par exemple à l'aide d'une seringue conventionnelle, par voie parentérale extra vasculaire, ou intramusculaire sous cutanée.

On entend par voie parentérale extra vasculaire toute voie de pénétration dans l'organisme autre que la voie digestive et la voie vasculaire (veines, artères et vaisseaux sanguins).

La composition de l'invention peut également être administrée par voie intra-oculaire, par voie intracavitale ou sur des prothèses préalablement à leur application, par voie vaginale, sur une plaie ouverte ou lors d'une intervention chirurgicale.

De nombreux documents décrivent des compositions pour usage topique contenant des organogels à base de lécithines (voir par exemple US N° 6 306 383). La lécithine est un mélange de phospholipides de faible poids moléculaire. Les lécithines sont amphotères, elles sont solubles dans l'alcool et elles forment une émulsion avec l'eau. Les organogels de lécithine ont été décrits comme véhicules utiles pour faciliter la pénétration des molécules à faible poids moléculaire (Willimann, H., et autres, " Organogel lécithine comme matrice pour le transport transdermique des médicaments", J. Pharm. Sci., vol. 81, 1992). Les organogels de lécithine sont obtenus en ajoutant un peu d'eau à une solution de lécithine dans des solvants organiques tel que le palmitate d'isopropyle ou le cyclooctane. Dans ces documents, l'eau est ajoutée pour former le gel désiré si bien que l'organogel est formé avant son application sur la peau.

Au contraire, les organogels de la présente invention sont sous forme liquide quand on les administre à un organisme vivant et prennent la forme de gel une fois qu'ils entrent en contact avec un liquide physiologique. Par ailleurs, les lécithines ne constituent pas des substances organogélatrices telles que définies dans le cadre de la présente invention.

On entend par liquide physiologique, tout liquide circulant dans un corps animal, tel que par exemple le liquide lymphatique, le liquide lacrymal, le liquide céphalo-rachidien, le liquide amniotique, le liquide parentéral et le sang.

L'organogel formé à partir de la composition selon l'invention possède des capacités de rétention de molécules bioactives et plus particulièrement de molécules d'un poids inférieur à 100 000 dalton présentant un caractère hydrophile, permettant d'envisager une libération desdites molécules dans l'organisme sur des périodes supérieures à 3 jours.

Enfin, ledit organogel formé dans l'organisme à partir de la composition selon l'invention a la capacité de s'éliminer lentement par érosion et/ou biodégradation progressive, sans toxicité pour l'organisme où il est implanté.

Cette propriété de gélification *in situ* conforme à l'invention est obtenue par l'utilisation d'un solvant organique hydrophobe, constituant la phase organique dudit organogel et par une substance organogélatrice (ou organogélateur), constituant la matrice solide dudit organogel.

Les molécules constituant cette substance organogélatrice, sont du type notamment des dérivés esters d'acides gras d'alanine qui ont la capacité de s'auto-assembler spontanément pour former une matrice immobilisant ledit liquide organique hydrophobe. Cet auto-assemblage moléculaire peut se réaliser par des liaisons hydrogène s'établissant entre les groupements de type alcool (-OH), acide (-COOH), amine (-NH ou NH2) portés par les molécules organogélatrices.

Plusieurs documents ont décrit l'utilisation de dérivés de L-alanine pour gélifier ou solidifier des liquides organiques non polaires, tels que les hydrocarbures. On peut citer par exemple la demande FR 2 281 162, ou bien Luo et al, et Bhattacharya et al (dans Chemical Communications, 2001). Les organogels formés n'étaient pas destinés à la libération prolongée de principes actifs dans un organisme vivant.

Par ailleurs, Hanabusa et al (dans Journal of Colloid and Interface Science, 1997), décrit la formation d'organogels utilisant le N-benzyloxycarbonyl-l-alanine 4-hexadecanoyl-2-nitrophenyl ester (BLAHN) ou le N-benzyloxycarbonyl-d-alanine 4-hexadecanoyl-2-nitrophenyl ester (BDAHN) comme substance organogélatrice.

Si nécessaire, la gélification de la composition liquide est induite par refroidissement du site d'application de la composition ou par diffusion d'un solvant organique hydrophile ajouté à la composition de l'invention.

La demanderesse a sélectionné des solvants organiques hydrophiles capables de créer des liaisons faibles (ex. : ponts hydrogène) avec les molécules de substance organogélatrice, et capables de diffuser dans les milieux aqueux pour réaliser la composition selon l'invention.

Ainsi, le solvant organique hydrophile, introduit dans le mélange formant la composition selon l'invention va entrer en compétition avec les molécules de substance organogélatrice, en créant avec lesdites molécules des liaisons faibles (ex. : ponts hydrogène) empêchant les dites molécules de s'auto-assembler en un réseau dense et uni. La composition selon l'invention restera donc sous forme liquide tant que les molécules dudit solvant organique hydrophile resteront liées aux molécules de l'organogélateur.

L'utilisation selon la présente invention de la réversibilité des liaisons faibles va avantageusement permettre à la matrice organogélatrice de se ré-assembler dès lors que ledit solvant organique hydrophile aura diffusé dans le milieu environnant.

Ainsi, dès son entrée en contact avec une solution aqueuse et plus particulièrement avec les liquides physiologiques tels que le liquide interstitiel, la lymphe ou le liquide intra péritonéal par exemple, ledit solvant organique hydrophile présent dans la composition conforme à l'invention va diffuser dans ledit liquide environnant du fait de son hydrophilie.

La diffusion dudit solvant organique hydrophile va alors permettre l'auto-assemblage des molécules de ladite substance organogélatrice. Cet auto-assemblage, en créant un réseau structuré, va permettre la rétention dudit liquide organique hydrophobe, faisant passer ladite composition de l'état liquide à l'état gel.

La présente invention offre donc un système simple de gélification *in situ* spontanée, et d'administration aisée.

Par ailleurs, la présente invention repose sur les propriétés d'hystérèse observées par la demanderesse sur des organogels à base de substances organogélatrices conformes à l'invention. On entend par hystérèse le phénomène physique observé notamment pour les compositions gélifiables, représentant l'écart existant entre la température de transition gel/liquide et la température de transition liquide/gel. Ces propriétés permettent en effet de concevoir une composition conforme à l'invention qui soit liquide, donc facilement injectable, à température ambiante (ou à une température avoisinant la température ambiante). De plus, ces propriétés permettent également de réaliser un organogel selon l'invention qui, une fois formé *in vivo,* va rester sous forme gel à la température corporelle de l'organisme considéré. En effet, un tel organogel, qu'il soit formé par diffusion ou simple refroidissement, possède une température de transition gel/liquide supérieure à la température du site d'injection ou d'application. De ce fait, il est parfaitement stable dans ledit organisme.

Enfin, la présente invention a l'avantage de fournir un support de libération prolongée de médicaments ou d'autres substances actives. En effet, l'organogel formé dans l'organisme et conforme à la présente invention comporte une véritable structure matricielle organisée qui a peu d'affinité pour le milieu aqueux environnant et permet donc une libération lente de la substance active par diffusion, érosion ou biodégradation progressive dudit organogel.

La présente invention fournit donc un support simple, efficace et facile d'administration permettant une libération prolongée dans l'organisme, d'une période au moins égale à 1 jour, de substances telles que des substances bioactives et plus particulièrement de molécules à caractère hydrophile d'un poids inférieur à 100 000 dalton.

En outre, la composition selon la présente invention a l'avantage d'être extrêmement peu coûteuse, tant sur le plan de la fabrication comme cela est décrit plus loin, que sur le plan du conditionnement et de l'administration.

Les substances organogélatrices conformes à l'invention sont des substances dont les molécules ont la capacité de se lier entre elles par liaisons de faible énergie, et notamment par liaisons hydrogène, permettant la formation d'une matrice thermosensible. Ces molécules sont le N-lauroyl L-alanine méthyle ester, le N-lauroyl L-alanine éthyle ester, le N-stéaroyl L-alanine méthyle ester, le N-stéaroyl L-alanine éthyle ester, et le N-lauroyl L-alanine acide.

Ces substances sont biocompatibles et ne donnent pas lieu à des métabolites toxiques ou dangereux pour l'organisme lors de leur dégradation par ce dernier.

Ce sont des dérivés d'alanine ou dérivés esters d'acides gras d'alanine, présentant à la fois une bonne biocompatibilité et un pouvoir organogélateur satisfaisant et surtout conférant au système gélifié des propriétés d'hystérèse. Ces propriétés se traduisent par un passage de l'état liquide à l'état gel à une température différente de celle observée lors du passage de la forme gel à la forme liquide de ladite composition. La demanderesse a le mérite d'avoir remarqué que l'écart entre ces deux températures de transition est variable en fonction du type de solvant organique hydrophobe utilisé, et de la quantité de substance organogélatrice utilisée.

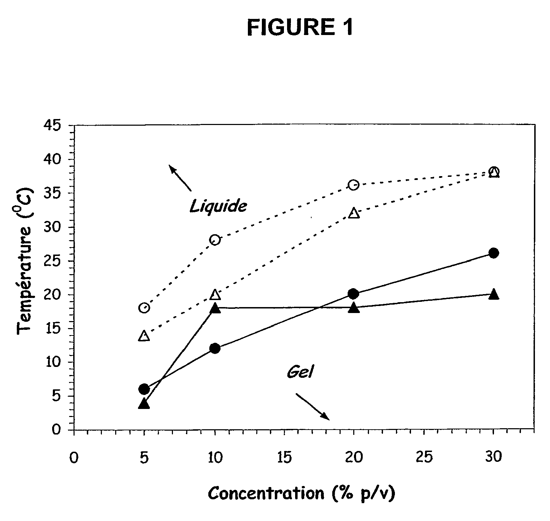

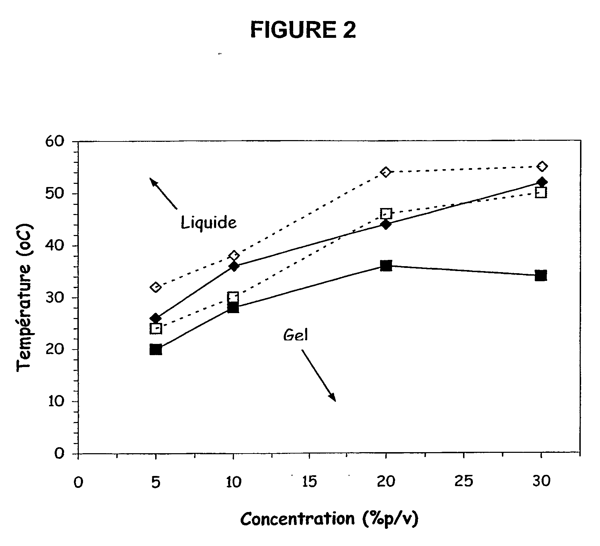

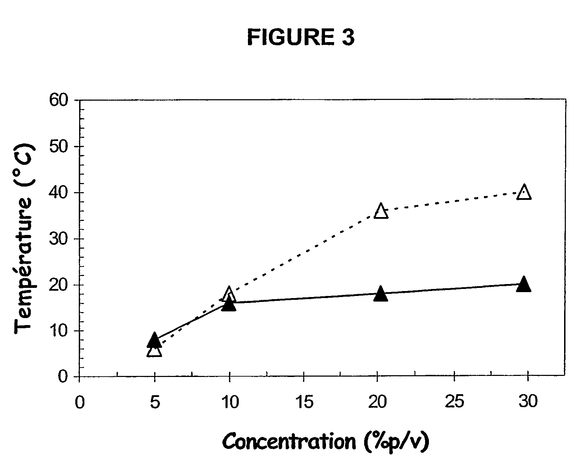

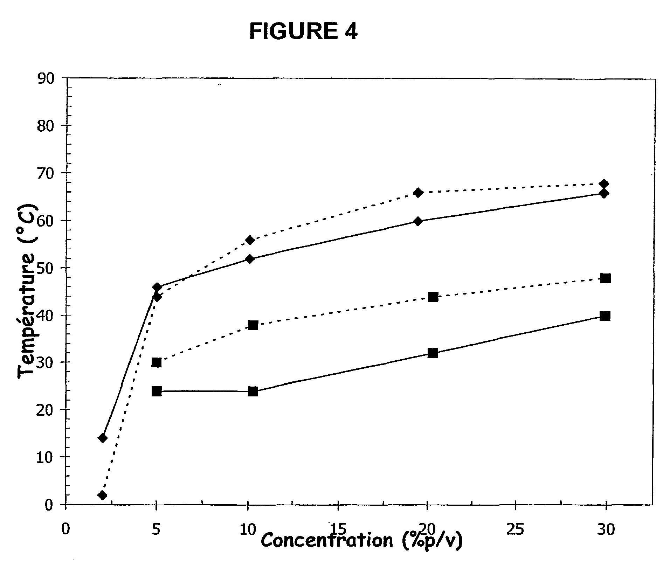

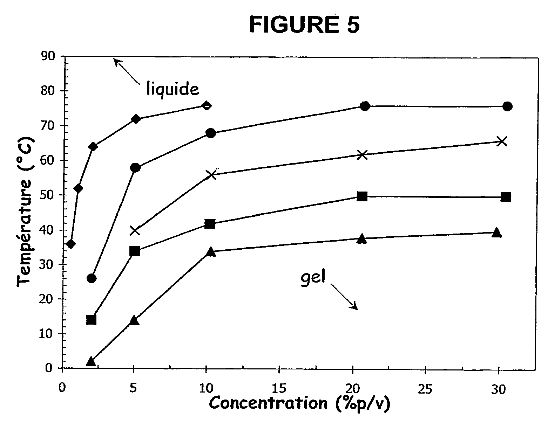

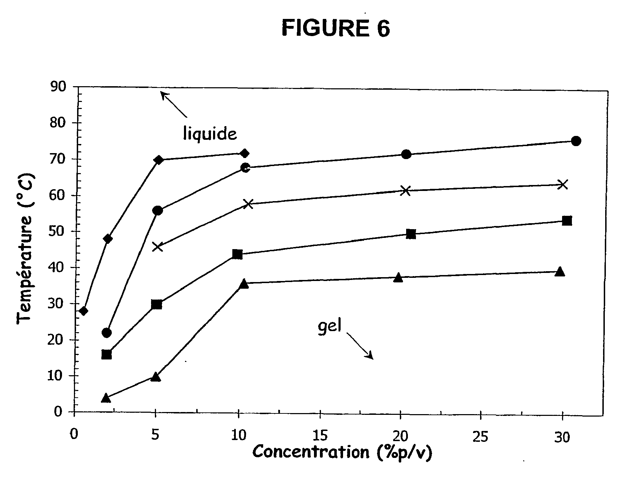

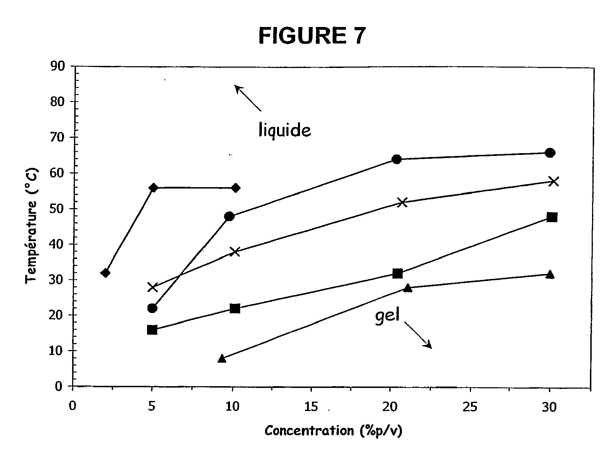

Ainsi, la demanderesse a réalisé des compositions conformes à l'invention (fig. 1-4) et des compositions à titre d'exemples comparatifs (fig. 5-7) dont les températures de transition et les écarts entre ces températures sont ajustables par simple modification de ces deux paramètres. Les résultats traduisant ces variations sont représentés sur les figures 1 à 7 (fig. 1-4, conformes à l'invention (fig. 5-7) à titre d'exemples comparatifs).

Préférentiellement, l'écart entre ces deux températures de transition est choisi pour que la température de transition liquide/gel soit inférieure à la température corporelle de l'organisme vivant considéré dans le cas où l'organogel est administré sans solvant organique hydrophile et que la température de transition gel/liquide soit supérieure à la susdite température.

Ainsi, on utilise les dérivé d'alanine N-lauroyl L-alanine acide (LA) ou des dérivés esters de l'alanine tels que le N-lauroyl L-alanine méthyle ester (LAM) ou le N-lauroyl L-alamine éthyle ester (LAE), le N-stéraroyl L-alanine méthyle ester (SAM) ou le N-stéaroyl L-alanine éthyle ester (SAE) comme substance organogélatrice conforme à l'invention.

La quantité de substance organogélatrice est fonction du type de liquide organique hydrophobe employé et de la température de transition qu'on souhaite choisir pour l'organogel conforme à l'invention.

Cependant, la proportion de cette substance est avantageusement choisie entre 0,5 et 50 % en poids du poids total de ladite composition.

La demanderesse a constaté que l'utilisation comme substance organogélatrice du N-lauroyl L-alanine méthyle ester permet à ladite composition de passer à l'état gélifié par simple refroidissement sous le seuil de transition liquide/gel et de demeurer à l'état gel à une température dépassant la température de transition liquide/gel en particulier la température de l'organisme vivant.

En effet, la demanderesse a remarqué que ledit organogel formé par refroidissement est stable dans l'intervalle de températures comprises entre la température de transition liquide/gel et la température de transition gel/liquide.

L'ensemble de ces constatations a conduit la demanderesse à élaborer une composition thermosensible à propriétés gélifiantes ayant la capacité de passer sous forme gélifiée par simple refroidissement local et de conserver cet état gélifié à la température corporelle. Dans ce cas particulier de l'invention, la quantité de solvant organique hydrophile peut être extrêmement réduite, voire nulle puisque la gélification s'opère par un refroidissement de ladite composition et non plus par diffusion dudit solvant organique hydrophile.

Ce mode de réalisation est particulièrement avantageux puisqu'il permet de s'affranchir de la présence du solvant organique hydrophile et donc de simplifier encore le procédé de préparation de la composition selon l'invention et également de diminuer son coût de revient.

Ainsi, selon un mode de réalisation particulier, la composition thermosensible selon l'invention contient une proportion de N-lauroyl L-alanine méthyle ester suffisante pour permettre le passage de ladite composition de l'état liquide à l'état d'organogel par simple refroidissement de ladite composition au contact de son site d'injection dans l'organisme.

Un tel refroidissement doit être suffisant pour faire passer ladite composition, appliquée sous forme liquide, à sa forme gélifiée. Ce refroidissement qui peut être opéré par apposition externe d'un objet froid tel qu'un pain de glace ou une compresse froide ou tout autre moyen refroidissant autour du site d'injection, doit permettre un abaissement local, sous la température de transition liquide/gel de ladite composition.

La composition selon l'invention est donc préférentiellement sous forme liquide à la température du site d'application, possède une température de transition gel/liquide supérieure à la température corporelle et une température de transition liquide/gel inférieure à la température de l'organisme considéré ou de la zone d'implantation du gel. En effet, la température cutanée peut être inférieure de quelques degrés à la température générale de l'organisme.

Dans un mode de réalisation préféré, la proportion en N-lauroyl L-alanine méthyle ester de ladite composition est suffisante pour que la température de transition liquide/gel soit inférieure à la température corporelle (37°C en général) et que la température de transition gel/liquide, soit supérieure à la température corporelle (37 °C en général).

D'une manière encore plus préférentielle, la composition selon l'invention doit comporter une température de transition liquide/gel inférieure à 30°C et une température de transition gel/liquide supérieure à + 35°C.

Ainsi, la composition selon l'invention est préférentiellement une composition dont l'intervalle entre la température de transition liquide/gel et la température de transition gel/liquide est avantageusement d'au moins 20°C, la température de transition liquide/gel étant préférentiellement comprise entre +5°C et + 36°C.

Le liquide organique hydrophobe de la composition selon la présente invention est un solvant organique hydrophobe ou un mélange de différents solvants organiques hydrophobes.

Les mélanges de différents solvants organiques hydrophobes présentent l'avantage de modifier le profil de gélification ou encore de faciliter la solubilisation de certaines substances bioactives.

Les solvants organiques hydrophobes utilisables pour la réalisation de la composition selon la présente invention appartiennent au groupe des solvants organiques non miscibles à l'eau capables de créer une structure de type organogel, en présence d'une quantité suffisante de substance dite organogélatrice telle que décrite ci-dessus.

Ces solvants sont biocompatibles, c'est-à-dire tolérés par l'organisme hôte, ne déclenchant pas ou peu de réaction immunitaire, de type inflammatoire ou allergique par exemple.

Enfin, on notera que l'on utilise des solvants organiques hydrophobes liquides à température ambiante ce qui simplifie le procédé de fabrication et d'administration de la composition conforme à l'invention.

On utilise des solvants organiques pouvant être dégradés de manière lente, c'est-à-dire, non rapidement métabolisés par les enzymes présentes sur le site d'injection, et notamment par les lipases.

Ainsi, les solvants organiques hydrophobes conformes à l'invention appartiennent au groupe comprenant les huiles végétales, les huiles semi-synthétiques et certains esters d'acides gras, notamment du glycérol (en particulier biglycérides et triglycérides)

On peut ainsi envisager l'utilisation de l'huile végétale biocompatible, l'huile de soja.

De façon préférentielle, on utilisera comme solvant organique hydrophobe l'huile végétale de soja présentant un comportement de gélification adéquat, une biodégradabilité lente et une excellente biocompatibilité.

Parmi les esters d'acides gras utilisables à titre de solvants organiques hydrophobes conformes à l'invention, on utilise des esters d'acides gras du glycérol, notamment les triglycérides. Plus préférentiellement, on utilisera les triglycérides à chaîne moyenne (inférieure à 18 atomes de carbone) tels que le Labrafac CC^{®} comportant deux acides gras de 8 et 10 atomes de carbone.

Parmi les solvants synthétiques ou semi-synthétiques utilisables comme solvants organiques hydrophobes conformément à la présente invention, on peut citer notamment le squalène, le benzoate de benzyle, le chlorure de benzyle, et les mélanges benzoate de benzyle/alcool benzylique. La présente description comprend en outre le Crodamol^{®} GTCC-PN.

On peut aussi combiner huiles et solvants organiques hydrophobes synthétiques.

On entend par solvant organique hydrophile selon l'invention, un solvant ayant une affinité importante pour les milieux aqueux, c'est-à-dire miscible à l'eau.

Le type de solvant organique hydrophile à utiliser dans la présente invention, est un solvant capable d'agir comme agent de déstabilisation de l'organogel, c'est-à-dire susceptible de créer des liaisons faibles avec les molécules d'organogélateur. Un tel solvant est par ailleurs avantageusement biocompatible, c'est-à-dire toléré par l'organisme, de telle sorte que sa diffusion n'entraîne pas ou peu de réaction immunitaire de type inflammatoire ou allergique. On utilisera donc de manière préférentielle pour la réalisation de la présente invention, un solvant ayant fait l'objet d'une approbation pour usage parentéral.

Ledit solvant organique hydrophile conforme à l'invention sera utilisé avantageusement dans des proportions inférieures à 60 % en poids de ladite composition, et plus préférentiellement inférieures à 20 %.

On peut citer parmi les solvants hydrophiles les solvants tels que les alcools comme l'éthanol, le glycérol, le propylène glycol, le poly(éthylène) glycol de faible poids moléculaire, l'alcool benzylique ou le chlorobutanol et leurs mélanges. Par ailleurs d'autres solvants miscibles à l'eau peuvent être envisagés, tels que le diméthyle sulfoxide (DMSO), le N-méthyl-pyrrolidone, le N-N-Diméthylacétamide, le furfural, le glycérol formal, l'isopropylidene glycérol, le lactate d'éthyle, l'acide acétique ou l'acide lactique et leurs mélanges.

Ces exemples ne sont pas limitatifs et on peut tout à fait concevoir de réaliser l'invention à partir d'autres composés organiques hydrophiles qui auraient des propriétés déstabilisatrices de gel, c'est-à-dire la capacité de créer des liaisons faibles avec la substance organogélatrice conforme à l'invention.

Les substances bioactives susceptibles d'être libérées dans l'organisme à partir de l'organogel conforme à la présente invention sont avantageusement des substances difficilement conditionnables pour une libération de façon prolongée telles que les molécules de faible poids moléculaire à caractère hydrophile ou très hydrophile. Avantageusement, ladite substance bioactive sera utilisée dans des proportions de 0,5 à 70 % en poids de la composition selon l'invention.

Ainsi, la demanderesse a testé le relargage à partir d'un organogel préformé de molécules de dextran marquées avec une molécule fluorescente : le FITC (Fluoro-Iso Thio Cyanate).

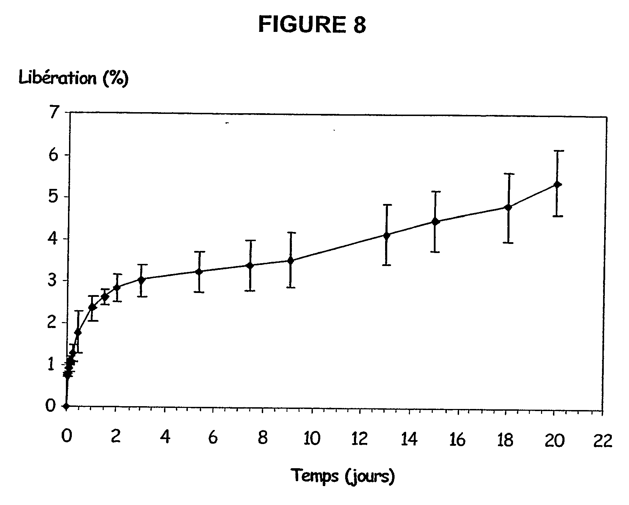

Le profil de libération du dextran a été suivi *in vitro* sur 20 jours par dosage de la fluorescence comme le montre la figure N° 8. La fluorescence a été mesurée par des prélèvements réguliers d'une solution aqueuse de tampon phosphate salin.

Ainsi, on peut concevoir la libération sur des périodes supérieures à 3 jours de protéines notamment d'intérêt thérapeutique telles que l'interféron α, l'interféron β, la somatostatine, la calcitonine, l'héparine, les interleukines ou l'érythropoïétine, de peptides, d'acides aminés ou de vitamines.

Ces exemples ne sont en aucun cas limitatifs et d'autres types de molécules, en particulier d'autres protéines peuvent tout à fait être envisagées pour une telle libération prolongée à partir d'un organogel conforme à l'invention.

On peut envisager le relargage dans l'organisme à partir de l'organogel selon l'invention, de molécules telles que certaines hormones et notamment certaines hormones peptidiques telles que l'hormone de croissance humaine, l'hormone thyréotrope ou le leuprolide.

Ainsi, on peut prévoir d'utiliser la présente invention pour la libération prolongée dans l'organisme d'acides nucléiques, d'oligonucléotides ou de dérivés d'acides nucléiques notamment.

De même, on peut tout à fait concevoir de réaliser la présente invention dans le but de solubiliser puis de libérer de façon prolongée des substances bioactives hydrophobes, c'est-à-dire présentant une forte affinité pour l'organogel et une faible affinité pour le milieu aqueux environnant.

La présente invention est donc utilisable pour un grand nombre de substances à intérêt thérapeutique ou médical pour lesquelles on souhaite une libération prolongée dans l'organisme.

A titre d'exemple, la composition selon la présente invention peut être préparée de la manière suivante.

### Cas d'un système de gélification par diffusion

On procède tout d'abord à la dissolution spontanée ou par chauffage et/ou agitation de l'organogélateur dans le solvant organique hydrophile.

Puis on incorpore la substance active et le (les) solvant(s) organique(s) hydrophobe(s) à ce mélange, deux cas peuvent alors se présenter :
a) Soit la substance active est soluble dans la phase organique ainsi formée :
   Dans ce cas, on solubilise la substance active dans la phase organique formée. La solubilisation s'opère spontanément ou par chauffage, avec ou sans agitation.
      On peut également prévoir dans ce cas la dissolution de ladite substance active directement dans le solvant organique hydrophobe.
b) Soit la substance active est peu ou pas soluble dans la phase organique :
   Dans ce cas, on procède tout d'abord au "mouillage" de la substance active en la dispersant dans la phase organique formée par le solvant organique hydrophile et la substance organogélatrice. Après agitation, il se forme alors une suspension de substance active dans le mélange. Cette suspension pourra alors être ajoutée aux autres constituants de la composition selon la présente invention.

Il est aussi possible de dissoudre la substance active dans une quantité juste suffisante d'eau. Cette solution aqueuse de substance active va être ajoutée à la phase organique formée par le solvant organique hydrophile et la substance organogélatrice. On procède ensuite à l'émulsion de cette phase aqueuse dans la phase organique par agitation vive. Plus vive est l'agitation, plus petite est la taille des particules aqueuses formées dans la suspension organique et plus stable est l'émulsion. Cette émulsion va ensuite pouvoir être utilisée pour la préparation d'une composition conforme à l'invention.

Il est à noter, toutefois, que cette technique utilisant une émulsion, a l'avantage de conserver les molécules complexes de substance active dans un micro environnement aqueux, ce qui limite beaucoup les perturbations dont elles peuvent faire l'objet lorsqu'elles sont soumises au changement d'environnement, en particulier les possibilités de dénaturation de la substance.

On ajoute ensuite au mélange précédemment obtenu le liquide organique hydrophobe, éventuellement sous agitation et/ou chauffage modéré jusqu'à obtenir un mélange homogène.

Ce mélange homogène selon l'invention peut alors être injecté dans un organisme vivant par voie parentérale extra vasculaire à l'aide d'une seringue conventionnelle pour injections sous cutanées. Après un temps de latence qui dépend de la formulation choisie, on assiste à la formation d'un durcissement sur le site d'injection, preuve de la formation *in vivo* de l'organogel selon l'invention.

S'il n'est pas extrait de manière chirurgicale, ledit organogel va, suivant sa taille et la nature des composants qui le constituent, être biodégradé et/ou s'éroder progressivement à plus ou moins longue échéance dans l'organisme. Cette biodégradation progressive va entraîner le relargage de la substance active éventuellement contenue dans l'organogel selon l'invention.

On choisira préférentiellement de réaliser de tels organogels dont la biodégradation sera comprise sur des périodes supérieures à 3 jours.

### Cas d'un système de gélification par refroidissement

Dans ce cas, on mélange tout d'abord la substance organogélatrice dotée de propriétés d'hystérèse avec le solvant organique hydrophobe.

Puis on procède à l'incorporation de la substance active dans ce mélange. Si la substance active est organosoluble, elle sera dissoute dans le mélange directement ou par faible agitation. Dans le cas où la substance active est peu ou pas organosoluble, on procède comme précédemment à la dispersion de cette dernière dans la phase organique ou à la réalisation d'une émulsion stable de substance active préalablement dissoute dans l'eau, dans la phase organique formée.

La composition ainsi formée est stable et préférentiellement liquide à la température ambiante. Elle est injectée par exemple par voie parentérale extravasculaire.

Immédiatement après l'injection, un objet froid (ou tout autre système de refroidissement) est maintenu au contact du site d'injection pendant une durée suffisante pour permettre la gélification *in situ* de la composition selon la présente invention.

Lorsque la gélification est accomplie, le système de refroidissement est retiré. Le site d'injection regagne alors la température corporelle, l'organogel selon l'invention restant stable à ladite température.

La composition thermosensible à propriétés gélifiantes conforme à l'invention peut être utilisée par exemple à la délivrance retard de substances bioactives sur de longues périodes, c'est-à-dire sur des périodes d'au moins un jour jusqu'à une semaine, généralement supérieures à 3 jours. Cette composition peut donc servir de support à la délivrance retard de tout type de substances, notamment de substances à intérêt thérapeutique ou médical.

On peut ainsi prévoir l'utilisation de la composition thermosensible conforme à l'invention pour la délivrance retard de médicaments nécessitant d'être maintenu à un taux sanguin constant. Cette invention se révèle donc particulièrement intéressante pour les médicaments administrés ordinairement par plusieurs prises quotidiennes destinées à maintenir un taux thérapeutique efficace dans l'organisme.

On peut ainsi envisager l'utilisation de l'invention pour des substances thérapeutiques telles que la morphine ou les médicaments agissant comme régulateurs du système cardio-vasculaire ou du système nerveux.

De même, on peut concevoir l'utilisation de la composition selon l'invention dans le but de pallier certaines carences de l'organisme, notamment en vitamines ou en hormones. Ainsi, la présente invention peut servir de support à la délivrance prolongée d'hormones nécessitant une prise quotidienne, et encore aujourd'hui administrées par injection, mode d'administration douloureux et contraignant. Un tel support de libération par organogel, de par son administration aisée, son innocuité et son faible coût permettrait de s'affranchir de ces contraintes pour le patient.

Cette composition peut également être utilisée pour la fabrication d'un médicament destiné à être injecté dans l'organisme par voie parentérale extravasculaire et notamment par voie sous cutanée, intradermique, intrapéritonéale ou intramusculaire, par voie intra-oculaire ou intravasculaire, par voie vaginale, sur une plaie ouverte ou lors d'une intervention chirurgicale.

Elle peut par ailleurs permettre la fabrication d'un médicament destiné à être utilisé comme vecteur de libération prolongée de substance(s) bioactive(s) dans l'organisme.

### FIGURES

La figure 1 représente le diagramme des températures de transition Liquide-gel (lignes pleines) et Gel-liquide (lignes pointillées) du N-lauroyl L-Alanine Méthyle ester (LAM) en présence de benzoate de benzyle (cercles) ou d'un mélange benzoate de benzyle/ alcool benzylique à 5 % (triangles).
La figure 2 représente le diagramme des températures de transition Liquide-gel (lignes pleines) et Gel-liquide (lignes pointillées) du LAM en présence d'huile de soja (losanges) ou de Labrafac^{®} CC (carrés).
La figure 3 représente le diagramme des températures de transition Liquide-gel (lignes pleines) et Gel-liquide (lignes pointillées) du N-lauroyl L-alanine éthyle ester (LAE) (triangles) en présence d'huile de soja.
La figure 4 représente le diagramme des températures de transition Liquide-gel (lignes pleines) et Gel-liquide (lignes pointillées) du LAM (carrés) et du N-stéaroyl L-alanine méthyle ester (SAM) (losanges) en présence d'oléate d'éthyle.
La figure 5 représente le diagramme des températures de transition Gel-liquide du LAM (carrés), LAE (triangles), SAM (cercles), N-stéaroyl L-alanine éthyle ester (SAE) (croix) et N-lauroyl L-alanine acide (LA) (losanges) dans l'huile de maïs.
Les figures 6 et 7 représentent les diagrammes des températures de transition Gel-liquide du LAM (carrés), LAE (triangles), SAM (cercles), SAE (croix) et LA (losanges) dans l'huile de carthame et le Crodamol® GTCC-PN (triglycérides) respectivement.
La figure 8 représente le suivi sur 20 jours du profil de libération in vitro du FITC-dextran (Poids moléculaire = 9500) à partir d'un gel constitué d'huile de soja et de 30 % de LAM dans le PBS à 37°C.
La figure 9 représente la photographie d'un implant conforme à l'exemple 5 au site d'injection.

### Exemple 1 : Formation d'un organogel in vivo à partir d'une composition conforme à l'invention.

Dans cet exemple, on cherche à vérifier que la composition conforme à l'invention est bien capable de gélifier *in vivo.* Les essais sont réalisés sur le rat.

On utilise comme solvant organique hydrophobe conforme à l'invention, l'huile de soja et l'éthanol comme solvant organique hydrophile selon l'invention.

La substance organogélatrice choisie est le LAM (N-lauroyl L-alanine méthyle ester)

Les proportions utilisées sont récapitulées dans le tableau suivant :

| **Produit** | **Fonction** | **Proportion** |
|---|---|---|
| LAM | Organogélateur | 20% p/v |
| Huile de soja | Solvant organique hydrophobe | qsp 100 mL |
| Ethanol | Solvant organique hydrophile | 14 % v/v |

On procède tout d'abord à la dissolution de l'organogélateur dans l'éthanol. Puis on rajoute à ce mélange l'huile de soja. Le mélange ainsi obtenu est agité et chauffé jusqu'à homogénéisation complète. Ce mélange reste stable et liquide à température ambiante.

On procède ensuite à l'injection sous cutanée de la composition ainsi obtenue. L'injection est pratiquée au niveau dorsal, à l'aide d'une seringue conventionnelle pour l'injection en sous cutané. Après 2 heures l'animal est sacrifié et un gel est extrait du site d'injection, démontrant la formation *in vivo* de l'organogel.

### Exemple 2 : Formation d'un organogel in vivo à partir d'une composition conforme à l'invention.

On utilise comme solvant organique hydrophobe conforme à l'invention, l'huile de soja et l'éthanol comme solvant organique hydrophile selon l'invention.

La substance organogélatrice choisie est le LAM (N-lauroyl, L-alanine méthyle ester)

Les proportions utilisées sont récapitulées dans le tableau suivant :

| **Produit** | **Fonction** | **Proportion** |
|---|---|---|
| LAM | Organogélateur | 30 % p/v |
| Huile de soja | Solvant organique hydrophobe | qsp 100 mL |
| Ethanol | Solvant organique hydrophile | 18% v/v |

Le procédé d'injection est identique à celui de l'exemple 1, de même que l'apparition d'un organogel 2h30 post-injection.

### Exemple 3 : Fabrication d'une composition selon l'invention contenant du FITC-Dextran

Cette composition permet de mesurer in vitro le relargage progressif d'une substance active contenue dans un organogel préformé.

On utilise comme principe actif le FITC-dextran qui va permettre de mesurer par dosage de la fluorescence associée, la quantité de dextran libérée par l'organogel conforme à l'invention.

Voir la figure 8.

Chaque point représente la valeur moyenne +/-sd (n=3). La surface du gel exposé était de 0.64 mm².

| **Produit** | **Fonction** | **Proportion** |
|---|---|---|
| FITC-Dextran | Substance active | 1,3 % p/p |
| LAM | Organogélateur | 30 % p/v |
| Huile de soja | Solvant organique hydrophobe | qp 100 mL |

On procède tout d'abord à la dissolution à chaud du LAM dans l'huile de soja. Puis on disperse à chaud le FITC-dextran dans la phase organique formée après l'avoir préalablement broyé au mortier, jusqu'à l'obtention d'une composition liquide homogène.

Ce mélange liquide est ensuite introduit gélifié par refroidissement dans un tube à essai. On ajoute sur le gel une solution aqueuse saline de tampon phosphate salin.

On prélève ensuite sur une période de 20 jours, des échantillons du liquide environnant l'organogel conforme à l'invention. On peut alors doser la fluorescence émise par ces échantillons et ainsi constater la libération prolongée du FITC-Dextran dans le milieu environnant. Dans l'hypothèse où un tel gel serait administré *in vivo* et conformément à la présente invention, un solvant organique du type éthanol devrait être rajouté de façon à inhiber le processus de gélification avant l'injection.

Les résultats de ce dosage sont récapitulés sur la figure N° 8

### Exemple 4 : Mise en évidence in vivo des propriétés hystérétiques d'un organogel conforme à l'invention. Exemple de gélification sans solvant hydrophile.

La solution organogélifiante est préparée à partir des constituants suivants :

| **Produit** | **Fonction** | **Proportion** |
|---|---|---|
| LAM | Organogélateur | 40 % p/v |
| Benzyl benzoate/5% alcool benzylique | Solvant organique hydrophobe | qsp 100 mL |

Les propriétés hystérétiques de cet organogel ont préalablement été déterminées pour que la température de transition liquide/gel soit inférieure à 30°C et que la température de transition gel/liquide soit supérieure à 37°C.

La solution organogélifiante est tout d'abord amenée à l'état liquide par chauffage, puis un volume de 180 µL de cette solution revenue à température ambiante est injecté en sous cutané chez le rat. Une fois l'injection terminée, une compresse à 4°C est apposée sur le site d'injection pendant 3 minutes, afin d'y abaisser la température et de provoquer la gélification.

L'animal est sacrifié 2h30 après l'injection et une observation visuelle de la forme de l'implant est effectuée. Puis l'implant est extrait du site d'injection et pesé.

Dans cette expérimentation, l'implant avait une forme discoïdale d'environ 1 cm de diamètre et un poids de 130 mg.

### Exemple 5 : Formation d'un organogel in vivo à partir d'une matrice constituée d'un mélange de solvants organiques hydrophobes.

Dans cet exemple, on souhaite vérifier la capacité de l'organogélateur à gélifier *in vivo* un organogel constitué d'un mélange de solvants organiques hydrophobes. Les essais sont réalisés chez le rat.

On utilise comme solvants organiques hydrophobes conformes à l'invention : l'huile de soja et l'oléate d'éthyle et l'éthanol comme solvant hydrophile.

La substance organogélatrice choisie est le LAM (N-lauroyl L-alanine méthyle ester).

La substance bioactive choisie est l'acétate de leuprolide. Une solution aqueuse d'acétate de Leuprolide à 0.67 %p/v est réalisée dans un premier temps.

Les proportions utilisées sont résumées dans le tableau suivant :

| **Produit** | **Fonction** | **Proportion** |
|---|---|---|
| LAM | Organogélateur | 20% p/v |
| Éthanol | Solvant organique hydrophile | 12% v/v |
| Solution d'acétate de leuprolide 0.67 %p/v | phase dispersée contenant la substance bioactive | 8% v/v |
| Huile de soja/Oléate d'éthyle (50:50 v/v) | solvant organique hydrophobe | qsp 100 mL |

On procède tout d'abord à la dissolution à chaud de l'organogélateur dans le mélange d'huile de soja et d'oléate d'éthyle. Puis on rajoute l'éthanol à ce mélange. Le mélange ainsi obtenu est agité et chauffé jusqu'à homogénéisation complète. Ce mélange reste stable et liquide à température ambiante. L'acétate de leuprolide est dissous dans l'eau distillée puis ajouté au mélange liquide. Ce mélange est agité puis émulsifié aux ultrasons pendant deux minutes.

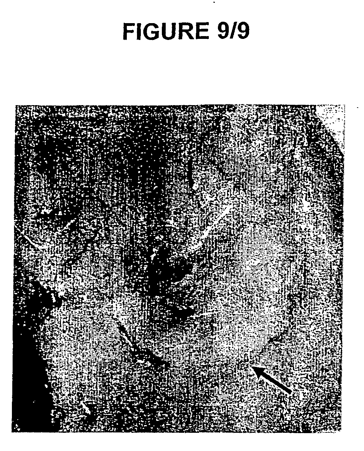

On procède ensuite à l'injection sous cutanée de la composition ainsi obtenue. L'injection est pratiquée au niveau dorsal chez le rat à l'aide d'une seringue conventionnelle pour l'injection en sous cutané. Après 2 heures, l'animal est sacrifié et un gel est extrait du site d'injection, démontrant la formation *in vivo* de l'organogel constitué d'un mélange de solvants organiques hydrophobes. La figure 9 présente la forme de l'implant, obtenu selon la composition décrite dans cet exemple, au site d'injection.

### Exemple 6 : Formation d'une évulsion contenant une substance bioactive à inclure dans la composition conforme à l'invention

On cherche à concevoir une émulsion stable d'eau dans l'huile (E/H) qui renferme une substance bioactive hydrophile telle l'acétate de leuprolide en solution dans la phase aqueuse (phase dispersée).

La substance bioactive choisie est l'acétate de leuprolide. Elle est dissoute dans l'eau dans une proportion de 7.62% p/v.

L'émulsion est stabilisée par deux tensioactifs, le polysorbate 20 (Tween 20) et le trioléate de sorbitan (Span 85), dont la proportion de chacun est ajustée en fonction de la balance hydrophile/hydrophobe de l'émulsion à concevoir.

Les proportions utilisées sont récapitulées dans le tableau suivant :

| **Produit** | **Proportion (v/v)** |
|---|---|
| Solution d'acétate de leuprolide 7.62% p/v | 12.2% |
| Solution à 10% p/v Tween 20 dans eau | 7.8% |
| Solution à 10% p/v Span 85 dans huile de soja | 2.2% |
| Huile de soja | 77.8% |

On prépare une solution à 10% p/v de Span 85 dans l'huile de soja et une solution de 10 %p/v de Tween 20 dans l'eau. Les différentes phases sont ensuite rassemblées et le mélange est agité et chauffé jusqu'à homogénéisation complète. Ce mélange reste stable et liquide à température ambiante et peut être ajouté directement à un mélange d'huiles, d'organogéléteur et de N,N-diméthylacétamide (DMAc) (voir exemple 7).

### Exemple 7 : Formation d'un organogal in vitro à partir d'une composition contenant une émulsion et un solvant organique hydrophile autre que l'éthanol

Dans cet exemple, on cherche à vérifier la capacité d'un solvant organique hydrophile autre que l'éthanol à inhiber la gélification de l'organogel à température ambiante.

On utilise comme solvant organique hydrophobe l'huile de soja et le N,N-diméthylacétamide (DMAc) comme solvant organique hydrophile.

La substance organogélatrice choisie est le LAM (N-lauroyl L-alanine méthyle ester).

Les proportions utilisées sont récapitulées dans le tableau suivant :

| **Produit** | **Fonction** | **Proportion** |
|---|---|---|
| LAM | Organogélateur | 18.4% p/v |
| N,N-diméthylacétamide | Solvant organique hydrophile | 18.4% v/v |
| Émulsion E/H | Véhicule de la substance bioactive | 8.2% v/v |
| Huile de soja | Solvant organique hydrophobe | qsp 100 mL |

On procède tout d'abord à la dissolution à chaud de l'organogélateur dans l'huile de soja. Puis on rajoute le DMAc à ce mélange. Le mélange ainsi obtenu est agité et chauffé puis l'émulsion est ajoutée et le mélange est agité jusqu'à homogénéisation complète. Ce mélange reste stable, et est visqueux à température ambiante (semblable à une crème) et peut être injecté comme tel à l'aide d'une seringue conventionnelle en sous cutané. Le gel se forme suite à l'injection de cette préparation visqueuse.

## Revendications

1. Composition liquide thermosensible à propriétés gélifiantes à titre de médicament injectable dans un organisme vivant par voie parentérale extravasculaire, intramusculaire, intradermique, intrapéritonéale, intra-oculaire, intracavitale, par voie vaginale, sur une plaie ouverte ou lors d'une intervention chirurgicale, contenant :
- un liquide organique hydrophobe, choisi parmi :
• l'huile de soja, le squalène, le benzoate de benzyle, le chlorure de benzyle, un mélange de benzoate de benzyle et d'alcool benzylique,
• les esters d'acides gras du glycérol,
• ou leur mélange,
- une substance organogélatrice choisie parmi : le N-lauroyl L-alanine méthyle ester, le N-lauroyl L-alanine éthyle ester, le N-stéaroyl L-alanine méthyle ester, le N-stéaroyl L-alanine éthyle ester, et le N-lauroyl L-alanine acide dont les molécules ont la capacité de se lier entre elles par liaisons de faible énergie, et
- une substance bioactive,
qui passe sous forme d'organogel lorsqu'elle entre en contact avec un liquide physiologique, lors de son administration à un corps animal, en particulier l'homme, ledit organogel restant sous forme de gel *in vivo* à la température corporelle.

2. Composition selon la revendication 1, **caractérisée en ce qu'**elle contient en outre un solvant organique hydrophile capable de créer des liaisons faibles avec la substance organogélatrice.

3. Composition selon l'une des revendications 1 ou 2, **caractérisée en ce que** la proportion du solvant organique hydrophile est inférieure à 60 %, et préférentiellement inférieure à 20 % en poids de ladite composition.

4. Composition selon l'une des revendications 2 à 3, **caractérisée en ce que** ledit solvant organique hydrophile appartient au groupe comprenant l'éthanol, le glycérol, l'alcool benzylique, le propylène glycol, le N-méthylpyrrolidone et le diméthylsulfoxide (DMSO), le poly (éthylène) glycol de faible poids moléculaire, le chlorobutanol, le furfural, le N-N-diméthyacétamide, le glycérol formal, l'isopropylidène glycérol, le lactate d'ethyle, l'acide acétique et l'acide lactique.

5. Composition selon la revendication 4, **caractérisée en ce que** ledit solvant organique hydrophile est l'éthanol.

6. Composition selon la revendication 1, **caractérisée en ce que** ledit mélange est un mélange d'huile de soja et d'oléate d'éthyle.

7. Composition selon l'une des revendications précédentes, **caractérisée en ce que** ladite substance biologiquement active appartient au groupe comprenant les protéines, les peptides, les acides aminés, les vitamines, les acides nucléiques et les oligonucléotides.

8. Composition selon la revendication 7, **caractérisée en ce que** ladite substance biologiquement active est choisie parmi la morphine, l'interféron α, l'interféron β, la somatostatine, l'héparine, les interleukines, l'érythropoïetine, la calcitonine, l'hormone de croissance humaine, l'hormone thyréotrope, le leuprolide.

9. Composition selon l'une des revendications précédentes, **caractérisée en ce que** la substance organogélatrice représente entre 0,5 et 50 % en poids du poids total de ladite composition.

10. Composition selon l'une des revendications 1 à 9, **caractérisée en ce qu'**elle est injectée dans un organisme vivant à l'aide d'une seringue pour injections sous-cutanées.

11. Composition selon la revendication 1, dans laquelle la formation dudit organogel se fait par refroidissement du site d'application de ladite composition.

12. Composition selon les revendications 1 à 11, **caractérisée en ce que** ledit organogel possède une température de transition de l'état liquide à l'état gel inférieure à la température du site d'application dans le cas où l'organogel est administré sans solvant organique hydrophile et une température de transition de l'état gel à l'état liquide supérieure à la température corporelle.

13. Composition selon l'une quelconque des revendications 1 à 12, **caractérisée en ce que** ledit organogel possède une température de transition de l'état liquide à l'état gel inférieure à 30 °C et une température de transition de l'état gel à l'état liquide supérieure à + 35°C.

14. Organogel obtenu à partir de la composition telle que définie dans l'une des revendications 1 à 13, **caractérisé en ce qu'**il reste sous forme gélifiée stable entre la température d'application et la température de transition gel/liquide de ladite composition.

15. Procédé de préparation d'une composition selon l'une des revendications 1 à 13, **caractérisé en ce que** la substance bioactive, éventuellement en solution aqueuse, est ajoutée au mélange constitué de la substance organogélatrice et du solvant organique hydrophobe.

16. Procédé de préparation d'une composition selon la revendication 2 qui consiste à
- dissoudre la substance organogélatrice dans le solvant organique hydrophile, puis à
- incorporer la substance bioactive et le solvant organique hydrophobe.

17. Procédé selon la revendication 16, **caractérisé en ce que** lorsque la substance bioactive est peu soluble ou pas soluble dans la phase organique, une solution aqueuse de ladite substance est dispersée sous agitation dans la phase organique constituée de la substance organogélatrice et du solvant organique hydrophile.

## Patentansprüche

1. Thermisch sensible flüssige Zusammensetzung mit gelbildenden Eigenschaften als in einen lebenden Organismus auf parenteralem extravaskulärem, intramuskulärem, intradermalem, intraperitonealem, intraokularem, intrakavitärem, vaginalem, über eine offene Wunde oder bei einem chirurgischen Eingriff injizierbares Arzneimittel, enthaltend:
- eine hydrophobe organische Flüssigkeit, ausgewählt aus:
• dem Sojaöl, dem Squalan, dem Benzylbenzoat, dem Benzylchlorid, einem Gemisch aus Benzylbenzoat und Benzylalkohol,
• den Fettsäureestern des Glycerols,
• oder ihrem Gemisch,
- eine Organogelatorsubstanz, ausgewählt aus: dem N-Lauroyl L-alaninmethylester, dem N-Lauroyl L-alaninethylester, dem N-Stearoyl L-alaninmethylester, dem N-Stearoyl L-alaninethylester und der N-Lauroyl L-alaninsäure, deren Moleküle die Fähigkeit besitzen, sich aneinander mittels schwachenergetischer Bindungen zu binden, und
- eine biologisch aktive Substanz,
die in Form eines Organogels wechselt, wenn sie bei ihrer Verabreichung in einen tierischen Körper, insbesondere des Menschen, mit einer physiologischen Flüssigkeit im Kontakt ist, wobei das Organogel bei Körpertemperatur in Form von *in vivo*-Gel bleibt.

2. Zusammensetzung nach Anspruch 1, **dadurch gekennzeichnet, dass** sie ferner ein hydrophiles organisches Lösungsmittel enthält, das imstande ist, mit der Organogelatorsubstanz schwache Bindungen zu bilden.

3. Zusammensetzung nach einem der Ansprüche 1 oder 2, **dadurch gekennzeichnet, dass** der Anteil des hydrophilen organischen Lösungsmittels unter 60 Gew.-% und vorzugsweise unter 20 Gew.-% der Zusammensetzung ist.

4. Zusammensetzung nach einem der Ansprüche 2 bis 3, **dadurch gekennzeichnet, dass** das hydrophile organische Lösungsmittel zu der Gruppe gehört, die das Ethanol, das Glycerol, den Benzylalkohol, das Propylenglycol, das N-Methylpyrrolidon und das Dimethylsulfoxid (DMSO), das Poly(ethylen)glycol mit geringem Molekulargewicht, das Chlorbutanol, das Furfural, das N-N-Dimethyacetamid, das Formalglycerol, das Isopropylidenglycerol, das Ethyllactat, die Essigsäure und die Milchsäure umfasst.

5. Zusammensetzung nach Anspruch 4, **dadurch gekennzeichnet, dass** das hydrophile organische Lösungsmittel Ethanol ist.

6. Zusammensetzung nach Anspruch 1, **dadurch gekennzeichnet, dass** das Gemisch ein Sojaöl- und Ethyloleatgemisch ist.

7. Zusammensetzung nach einem der vorangehenden Ansprüche, **dadurch gekennzeichnet, dass** die biologisch aktive Substanz zu der Gruppe gehört, die die Proteine, die Peptide, die Aminosäuren, die Vitamine, die Nukleinsäuren und die Oligonukleotide umfasst.

8. Zusammensetzung nach Anspruch 7, **dadurch gekennzeichnet, dass** die biologisch aktive Substanz aus dem Morphin, dem Interferon α, dem Interferon β, dem Somatostatin, dem Heparin, den Interleukinen, dem Erythropoietin, dem Calcitonin, dem menschlichen Wachstumshormon, dem thyreotropen Hormon, dem Leuprolid ausgewählt ist.

9. Zusammensetzung nach einem der vorangehenden Ansprüche, **dadurch gekennzeichnet, dass** die Organogelatorsubstanz zwischen 0,5 und 50 Gew.-% des Gesamtgewichts der Zusammensetzung darstellt.

10. Zusammensetzung nach einem der Ansprüche 1 bis 9, **dadurch gekennzeichnet, dass** sie in einen lebenden Organismus mit Hilfe einer Spritze für subkutane Injektionen injiziert wird.

11. Zusammensetzung nach Anspruch 1, wobei die Bildung des Organogels durch Abkühlen der Applikationsstelle der Zusammensetzung erfolgt.

12. Zusammensetzung nach den Ansprüchen 1 bis 11, **dadurch gekennzeichnet, dass** das Organogel eine Übergangstemperatur vom flüssigen Zustand in den Gelzustand unter der Temperatur der Applikationsstelle besitzt in dem Fall, wenn das Organogel ohne hydrophiles organisches Lösungsmittel verabreicht wird, und eine Übergangstemperatur vom Gelzustand in den flüssigen Zustand über der Körpertemperatur.

13. Zusammensetzung nach einem der Ansprüche 1 bis 12, **dadurch gekennzeichnet, dass** das Organogel eine Übergangstemperatur vom flüssigen Zustand in den Gelzustand unter 30 °C besitzt und eine Übergangstemperatur vom Gelzustand in den flüssigen Zustand über +35 °C.

14. Organogel, hergestellt aus der Zusammensetzung nach einem der Ansprüche 1 bis 13, **dadurch gekennzeichnet, dass** es zwischen der Applikationstemperatur und der Gel-Flüssigkeits-Übergangstemperatur der Zusammensetzung in stabiler gelierter Form bleibt.

15. Herstellungsverfahren einer Zusammensetzung nach einem der Ansprüche 1 bis 13, **dadurch gekennzeichnet, dass** die biologisch aktive Substanz, eventuell in wässriger Lösung, dem Gemisch hinzugefügt wird, das aus der Organogelatorsubstanz und dem hydrophoben organischen Lösungsmittel gebildet ist.

16. Herstellungsverfahren einer Zusammensetzung nach Anspruch 2, das darin besteht:
- die Organogelatorsubstanz in dem hydrophilen organischen Lösungsmittel zu lösen und dann
- die biologisch aktive Substanz und das hydrophobe organische Lösungsmittel einzuarbeiten.

17. Verfahren nach Anspruch 16, **dadurch gekennzeichnet, dass**, wenn die biologisch aktive Substanz in der organischen Phase schwach löslich oder nicht löslich ist, eine wässrige Lösung der Substanz unter Rühren in der organischen Phase dispergiert wird, die aus der Organogelatorsubstanz und dem hydrophilen organischen Lösungsmittel besteht.

## Claims

1. Heat-sensitive liquid composition with gelling properties as medicinal product injectable into a living organism via the extravascular parenteral, intramuscular, intradermal, intraperitoneal, intraocular or intracavitary route, via the vaginal route, on an open wound or during surgery, containing:
- a hydrophobic organic liquid, selected from:
• soybean oil, squalene, benzyl benzoate, benzyl chloride, a mixture of benzyl benzoate and benzyl alcohol,
• fatty acid esters of glycerol,
• or mixtures thereof,
- an organogelling substance selected from: N-lauroyl-L-alanine methyl ester, N-lauroyl-L-alanine ethyl ester, N-stearoyl-L-alanine methyl ester, N-stearoyl-L-alanine ethyl ester, and N-lauroyl-L-alanine acid the molecules of which have the capacity to bind to each other via low-energy bonds, and
- a bioactive substance,
which changes to the organogel form when it comes into contact with a physiological fluid, during its administration into an animal body, in particular humans, said organogel remaining in gel form *in vivo* at body temperature.

2. Composition according to claim 1, **characterized in that** it further contains a hydrophilic organic solvent capable of creating weak bonds with the organogelling substance.

3. Composition according to one of claims 1 or 2, **characterized in that** the proportion of the hydrophilic organic solvent is less than 60%, and preferably less than 20% by weight of said composition.

4. Composition according to one of claims 2 to 3, **characterized in that** said hydrophilic organic solvent belongs to the group comprising ethanol, glycerol, benzyl alcohol, propylene glycol, N-methylpyrrolidone and dimethylsulfoxide (DMSO), low molecular weight poly(ethylene) glycol, chlorobutanol, furfural, N,N-dimethyacetamide, glycerol formal, isopropylidene glycerol, ethyl lactate, acetic acid and lactic acid.

5. Composition according to claim 4, **characterized in that** said hydrophilic organic solvent is ethanol.

6. Composition according to claim 1, **characterized in that** said mixture is a mixture of soybean oil and ethyl oleate.

7. Composition according to one of the preceding claims, **characterized in that** said biologically active substance belongs to the group comprisingproteins, peptides, amino acids, vitamins, nucleic acids and oligonucleotides.

8. Composition according to claim 7, **characterized in that** said biologically active substance is selected from morphine, α-interferon, β-interferon, somatostatin, heparin, interleukins, erythropoietin, calcitonin, human growth hormone, thyrotropin-releasing hormone, leuprolide.

9. Composition according to one of the preceding claims, **characterized in that** the organogelling substance represents between 0.5 and 50% by weight of the total weight of said composition.

10. Composition according to one of claims 1 to 9, **characterized in that** it is injected into a living organism using a syringe for subcutaneous injections.

11. Composition according to claim 1, wherein said organogel is formed by cooling the site of application of said composition.

12. Composition according to claims 1 to 11, **characterized in that** said organogel has a transition temperature from the liquid state to the gel state which is lower than the temperature of the site of application if the organogel is administered without hydrophilic organic solvent and a transition temperature from the gel state to the liquid state which is higher than body temperature.

13. Composition according to any one of claims 1 to 12, **characterized in that** said organogel has a transition temperature from the liquid state to the gel state lower than 30°C and a transition temperature from the gel state to the liquid state higher than +35°C.

14. Organogel obtained from the composition defined in one of claims 1 to 13, **characterized in that** it remains in stable gelled form between the application temperature and the gel/liquid transition temperature of said composition.

15. Process for preparing a composition according to one of claims 1 to 13, **characterized in that** the bioactive substance, optionally in aqueous solution, is added to a mixture consisting of the organogelling substance and the hydrophobic organic solvent.

16. Process for preparing a composition according to claim 2 which consists in
- dissolving the organogelling substance in the hydrophilic organic solvent, then in
- incorporating the bioactive substance and the hydrophobic organic solvent.

17. Process according to claim 16, **characterized in that** when the bioactive substance is poorly soluble or not soluble in the organic phase, an aqueous solution of said substance is dispersed with stirring in the organic phase consisting of the organogelling substance and the hydrophilic organic solvent.
